# EUROPEAN PATENT SPECIFICATION

(11) **EP 1 703 934 B1**
(45) Date of publication and mention of the grant of the patent: **14.09.2011**
(21) Application number: 04813283.1
(22) Date of filing: 06.12.2004
(51) Int. Cl.: A61M 25/00

(54) **CATHETER INCORPORATING AN IMPROVED POLYMER SHAFT**
KATHETER MIT OPTIMIERTEM POLYMERSCHAFT
CATHETER A TIGE POLYMERE INTEGREE, AMELIOREE

(30) Priority: 29.12.2003 US 750586
(43) Date of publication of application: 27.09.2006
(73) Proprietor: Boston Scientific Limited, St. Michael (BB)
(72) Inventor: WILLARD, Martin R., Burnsville, Minnesota 55337 (US); PU, Zhou, Maple Grove, MN 55311 (US)
(74) Representative: Vossius & Partner
(86) International application number: PCT/US2004/040948
(87) International publication number: WO 2005/065760

(56) References cited:
- EP-A- 0 594 201
- US-A- 5 336 205
- US-A- 5 658 263
- US-A1- 2003 167 051

## Description

### Field of the Invention

The present invention generally relates to intravascular medical devices. More particularly, the present invention relates to intravascular catheters having improved polymer blend catheter shafts. See for example US 2003/0167051.

### Background of the Invention

Diagnostic catheters and guide catheters are commonly used to facilitate the diagnosis and treatment of vascular diseases such as coronary artery disease and peripheral vascular disease. Balloon catheters are commonly used to treat vascular disease by dilating stenotic lesions. Because such intravascular catheters must be navigated to remote vascular sites through vascular anatomy that may be very tortuous, it may be desirable for the catheter shaft to exhibit a certain characteristic such as torqueability, trackability and pushability. A number of catheter shafts have been developed with these characteristics. Each has certain advantages and disadvantages. There is an ongoing need to provide alternative designs and methods for making and using catheter shaft with desirable characteristics and features.

### Summary of the Invention

The invention provides design, material, and manufacturing method alternatives for medical devices, for example, catheter shafts. An example catheter shaft may include a polymer blend. The polymer blend may include polyoxymethylene blended with a polymer having an ether group, for example, polyether polyester. In some embodiments, the shaft may include a proximal portion, an intermediate portion, and a distal portion. Each portion may have the same or differing proportions of polyoxymethylene. The shaft may also include an inner layer and an outer layer, each of which may have the same or differing proportions of polyoxymethylene. These and some of the other features and characteristics of suitable catheter shafts are described in more detail below.

The above summary of some embodiments is not intended to describe each disclosed embodiment or every implementation of the present invention. The Figures, and Detailed Description which follow more particularly exemplify these embodiments.

### Brief Description of the Drawings

The invention may be more completely understood in consideration of the following detailed description of various embodiments of the invention in connection with the accompanying drawings in which:
Figure 1 is a plan view of an example catheter;
Figure 2 is a cross-sectional view of the catheter of Figure 1 taken along line 2-2;
Figure 3 is a longitudinal sectional view of the catheter of Figure 1 taken along line 3-3;
Figure 4 is a plan view of another example catheter, shown as a balloon catheter; and
Figure 5 is a cross-sectional view of the catheter of Figure 4 taken along line 5-5.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

### Detailed Description of the Invention

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

Weight percent, percent by weight, wt%, wt-%, % by weight, and the like are synonyms that refer to the concentration of a substance as the weight of that substance divided by the weight of the composition and multiplied by 100.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

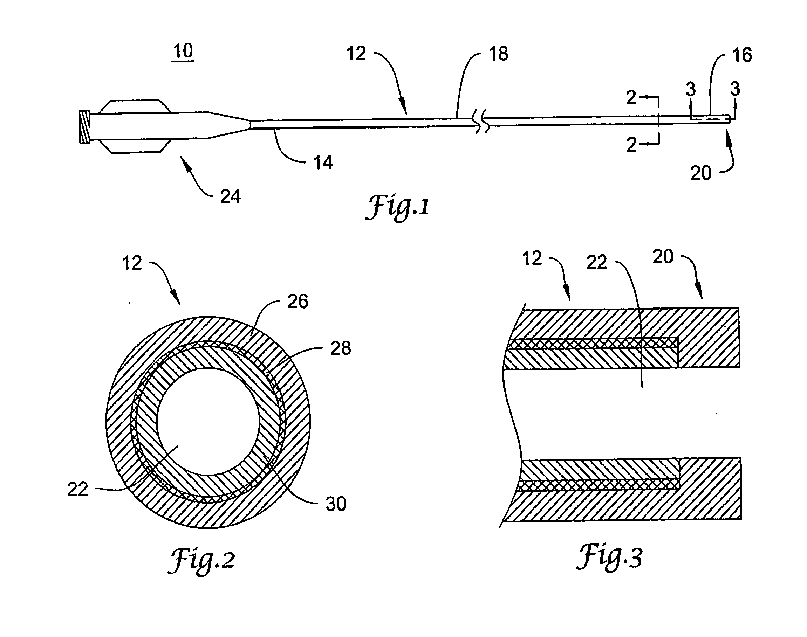

Figure 1 illustrates an example embodiment of a catheter 10. Catheter 10 may include a catheter shaft 12 having a proximal region 14, a distal region 16, an intermediate region 18 disposed between the proximal and distal regions 14/16, and a distal tip 20 disposed adjacent distal region 16. Distal region 16, intermediate region 18, distal tip 20, or any other suitable region of shaft 12 may be curved depending on the particular clinical application. Shaft 12 may include a lumen 22 (best seen in Figure 2) extending therethrough, which may be used, for example, to facilitate insertion of other medical devices (e.g., guidewires, balloon catheters, etc.) therethrough, and/or to facilitate injection of fluids (e.g., radiopaque dye, saline, drugs, etc.) therethrough. A manifold 24 can be connected to proximal section 14 of shaft 12 to facilitate connection to other medical devices (e.g., syringe, Y-adapter, etc.) and to provide access to the lumen 22. For purposes of illustration and discussion only, the intravascular catheter shown in Figure 1 is in the form of a guide or diagnostic catheter 10, but may comprise virtually any catheter used for intravascular applications including balloon catheters, micro catheters, and the like.

Catheter 10 may have a length and an outside diameter sufficient to enable intravascular insertion and navigation. For example, catheter 10 may have a length of approximately 100 cm to 150 cm and an outside diameter of approximately 4 to 9 French. The different sections of catheter may have various lengths. For example, proximal section 14 of shaft 12 may be from about 60 to about 135 cm or about 60 to about 90 % of the total length. Intermediate section 18 of shaft 12 may be from about 15 to about 30 cm or about 15 to about 20 % of the total length. Distal section 16 of shaft 12 may be from about 2 to about 10 cm or about 2 to about 7 % of the total length.

Shaft 12, or sections thereof, may be manufactured from or otherwise include a polymer blend. The polymer blend generally includes polyoxymethylene blended with a polymer having an ether group or moiety. For example, the polymer blend may include polyoxymethylene blended with a polyether polyester such as ARNITEL® available from DSM Engineering Plastics or HYTREL® available from DuPont. Other suitable polymers that may be blended with polyoxymethylene include polyether block ester, polyether block amide (PEBA, for example available under the trade name PEBAX®), polyetheretherketone (PEEK), polyetherimide (PEI), and the like. A suitable polyoxymethylene is commercially available under the trade name Delrin™ commercially available from DuPont Wilmington, DE.

Proximal section 14, intermediate section 18 and distal section 16 may each be formed with the same polymer blend. Alternatively, each section may be made from a different blend. The different blends may have differing amounts of polyoxymethylene and polyether polyester. For example, proximal section 14 may have about 80 to about 95 weight % polyoxymethylene and about 5 to about 20 weight % polyether polyester. Intermediate section 18 can have about 20 to about 50 weight % polyoxymethylene and about 50 to about 80 weight % polyether polyester. Distal section 16 can have about 5 to about 20 weight % polyoxymethylene and about 80 to about 95 weight % polyether polyester. In an alternative embodiment, distal section 16 can include about 0 to about 5 weight % polyoxymethylene and about 95 to about 100 weight % polyether polyester.

Proximal section 14, intermediate section 18 and distal section 16 may each have a different flexural modulus. For example, proximal section 14 can have a flexural modulus of about 210 to about 380 ksi. Intermediate section 18 can have a flexural modulus of about 30 to about 90 ksi. Distal section 16 can have a flexural modulus of less than about 30 ksi or from about 1 to about 30 ksi or from about 15 to about 30 ksi. The differences in flexural modulus can be varied, for example, by altering the proportion of polyoxymethylene and polyether polyester. For example, the flexural modulus can be decreased by increasing the amount of polyether polyester and/or decreasing the amount of polyoxymethylene. It can be appreciated that variations in flexural modulus can be made .

Manufacturing the polymer blended sections 14/16/18 may include extrusion of a polyoxymethylene pre-blend or by co-extrusion of the polyoxymethylene with the polyether polyester such as by interrupted layer co-extrusion (ILC). Alternatively, the sections 14/16/18 may be formed of separate extruded tubular segments subsequently fused together. In some embodiments, shaft 12 (i.e., sections 14/16/18) may include a single layer of polymer blend. According to these embodiments, the polymer blend can be extruded over a suitable die or mandrel. It can be appreciated that a number of other known manufacturing methods may be utilized without departing from the spirit of the invention.

In other embodiments, sections 14/16/18 may have a multi-layer construction. For example, shaft 12 may include a polymer blend outer layer 26, a reinforcement layer 28 and an inner layer 30 as shown in Figure 2. Outer layer 26 may generally span sections 14/16/18 and be made from a polymer blend as described above. For example, outer layer 26 may have may have about 80 to about 95 weight % polyoxymethylene and about 5 to about 20 weight % polyether polyester adjacent proximal section 14, about 20 to about 50 weight % polyoxymethylene and about 50 to about 80 weight % polyether polyester adjacent intermediate section 18, and about 5 to about 20 weight % polyoxymethylene and about 80 to about 95 weight % polyether polyester adjacent distal section 16. Moreover, outer layer 26 may be manufactured utilizing the extrusion techniques described above.

Reinforcement layer 28 may comprise a braid, coil, or other suitable reinforcing structure. Reinforcement layer 28 may be made from a number of different materials including metals, metal alloys, polymers, metal-polymer composites, and the like, or any other suitable material. Some examples of suitable metals and metal alloys include stainless steel, such as 304V, 304L, and 316L stainless steel; nickel-titanium alloy such as linear-elastic or super-elastic nitinol, nickel-chromium alloy, nickel-chromium-iron alloy, cobalt alloy, tungsten or tungsten alloys, MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si), hastelloy, monel 400, inconel 825, or the like; or other suitable material.

In some embodiments, reinforcement layer 28 may be made from or otherwise include a radiopaque material. Radiopaque materials are understood to be materials capable of producing a relatively bright image on a fluoroscopy screen or another imaging technique during a medical procedure. This relatively bright image aids the user of catheter 10 in determining its location. Some examples of radiopaque materials can include, but are not limited to, gold, platinum, palladium, tantalum, tungsten alloy, plastic material loaded with a radiopaque filler, and the like.

Inner layer 30 may include a lubricious, a hydrophilic, a protective, or other type of material or coating. Hydrophobic materials or coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609 . Alternatively, inner layer 30 may comprise a polyoxymethylene homopolymer or a polyoxymethylene blend as discussed herein.

Distal tip 20 may have a number of different forms or configurations. For example, distal tip 20 may be defined by a region where outer layer 26 extends beyond inner layer 30 and reinforcement layer 28 to define a soft atraumatic tip. In some embodiments, distal tip 20 may have essentially the same material composition as the adjacent portion of outer layer 26. For example, distal tip 20 includes a polymer blend having about 5 to about 20 weight % polyoxymethylene and about 80 to about 95 weight % polyether polyester. Alternatively, distal tip 20 may be made from a different material and fused to or co-extruded with outer layer 26. For example, distal tip 20 may be made from polyether polyester or another suitable (e.g., "soft") polymer.

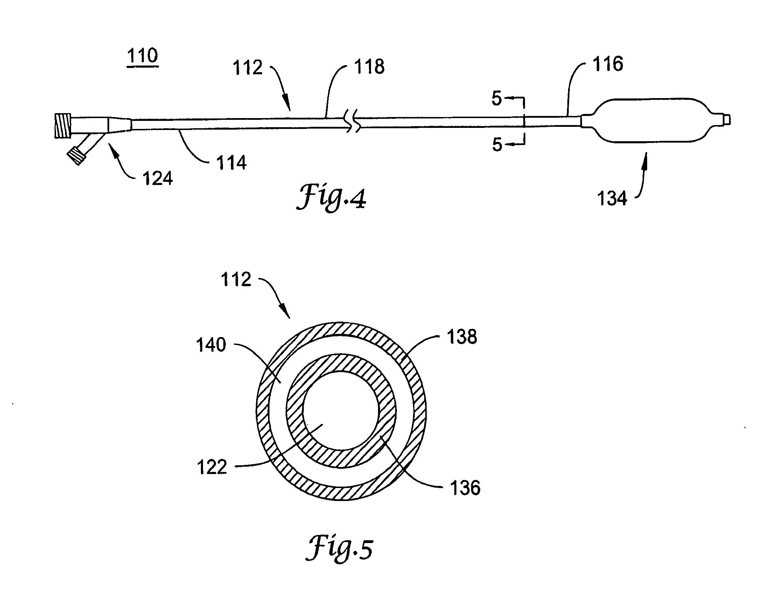

Figure 4 illustrates another example catheter 110 in the form of an intravascular balloon catheter. Catheter 110 includes shaft 112 having proximal portion 114, distal portion 116, and intermediate portion 118 disposed between proximal portion 114 and distal portion 116. An inflatable balloon 134 is connected to distal portion 118 of shaft 112. Depending on the type (over-the-wire, fixed-wire, single-operator-exchange, etc.) of balloon catheter 110, all or a portion of shaft 112 may include an inner tube 136 defining a guidewire lumen 122 therein, and an outer tube 138 disposed thereon to define an annular inflation lumen 140 therebetween. A manifold 124 can be connected to the proximal end of proximal portion 114 of shaft 112 to facilitate connection to other medical devices (e.g., syringe, Y-adapter, etc.) and to provide access to lumen 122 and/or 140.

Inner tube 136 may comprise a lubricious polymer similar to those describe above in relation to inner layer 30. For example, inner tube 136 may comprise HDPE or PTFE. Outer tube 138 may comprise a polymer blend that is similar to or the same as the polymer blends of the outer layer 26 discussed above. In addition, the manufacture and arrangement of parts for outer tube 138 may be similar to or the same as that discussed with reference to the outer layer 26.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A catheter shaft (20), comprising:
an inner layer (30);
a support member disposed over the inner layer (30); and
an outer layer (26) disposed over the inner layer (30) and the support layer, the outer layer comprising:
a proximal section having about 80 to about 95 weight % polyoxymethylene blended with about 5 to 20 weight % of a polymer having an ether group;
a distal section having about 5 to about 20 weight % polyoxymethylene and about 80 to about 95 weight % of a polymer having an ether group;
an intermediate section disposed between the proximal section and the distal section, the intermediate section having about 20 to about 50 weight % polyoxymethylene and about 50 to about 80 weight % of a polymer having an ether group.

2. The catheter shaft of claim 1, wherein the polymer having an ether group is polyether polyester.

3. The catheter shaft of either of claims 1 or 2, wherein the proximal section has a flexural modulus of about 210 to about 380 ksi;
the intermediate section has a flexural modulus of about 30 to 90 ksi; and
the distal section has a flexural modulus of less than 30 ksi.

4. The catheter shaft of either of claims 2 or 3, wherein the proximal section, intennediate section and distal section define a total shaft length and the proximal section is about 60 to about 90% of the total length, the intermediate section is about 15 to about 20% of the total length, and the distal section is about 2 to about 7% of the total length.

5. The catheter shaft of either of claims 2 or 3, further comprising a distal tip coupled to the distal section of the catheter shaft.

6. The catheter shaft of claim 5, wherein the distal tip is comprised of polyether polyester.

7. The catheter shaft of claim 2, wherein the inner layer includes polytetrafluoroethylene.

8. The catheter shaft of either of claims 1 or 2, wherein the inner layer includes high density polyethylene.

9. The catheter shaft of either of claims 1 or 2, wherein the support member includes a metallic braid.

10. The catheter shaft of either of claims 1 or 2, wherein the support member includes a coil.

11. The catheter shaft of either of claims 1 or 2, further comprising a distal tip coupled to and disposed distally of the inner layer, first layer, and support member.

12. The catheter shaft of either of claims 1 or 2, wherein the inner layer defines a guidewire lumen extending therethrough.

13. The catheter shaft of either of claims 1 or 2, wherein an inflation lumen is defined between the inner layer and the first layer.

14. A method for manufacturing a catheter shaft according to claim 1, comprising the steps of:
providing a first polymer blend having about 80 to about 95 weight % polyoxymethylene and about 5 to about 20 weight % polyether polyester;
providing a second polymer blend having about 20 to about 50 weight % polyoxymethylene and about 50 to about 80 weight % polyether polyester; and
providing a third polymer blend having about 5 to about 20 weight % polyoxymethylene and about 80 to about 95 weight % polyether polyester;
extruding the first polymer blend to define a first shaft member;
extruding the second polymer blend to define a second shaft member;
extruding the third polymer blend to define a third shaft member; and
coupling the first shaft member, second shaft member, and third shaft member to define a catheter shaft.

## Patentansprüche

1. Katheterschaft (20), der aufweist:
eine Innenschicht (30);
ein Stützelement, das über der Innenschicht (30) angeordnet ist; und
eine Außenschicht (26), die über der Innenschicht (30) und
der Stützschicht angeordnet ist, wobei die Außenschicht aufweist:
einen proximalen Abschnitt, der etwa 80 bis etwa 95 Gew.-% Polyoxymethylen aufweist, das mit etwa 5 bis 20 Gew.-% eines Polymers gemischt ist, das eine Ethergruppe aufweist;
einen distalen Abschnitt, der etwa 5 bis etwa 20 Gew.-% Polyoxymethylen und etwa 80 bis etwa 95 Gew.-% eines Polymers aufweist, das eine Ethergruppe aufweist;
einen Zwischenabschnitt, der zwischen dem proximalen Abschnitt und dem distalen Abschnitt angeordnet ist, wobei der Zwischenabschnitt etwa 20 bis etwa 50 Gew.-% Polyoxymethylen und etwa 50 bis etwa 80 Gew.-% eines Polymers aufweist, das eine Ethergruppe aufweist.

2. Katheterschaft nach Anspruch 1, wobei das Polymer, das eine Ethergruppe aufweist, Polyetherpolyester ist.

3. Katheterschaft nach einem der Ansprüche 1 oder 2, wobei der proximale Abschnitt ein Biegemodul von etwa 210 bis etwa 380 ksi aufweist;
der Zwischenabschnitt ein Biegemodul von etwa 30 bis 90 ksi aufweist; und
der distale Abschnitt ein Biegemodul von weniger als 30 ksi aufweist.

4. Katheterschaft nach einem der Ansprüche 2 oder 3, wobei der proximale Abschnitt, der Zwischenabschnitt und der distale Abschnitt eine Schaftgesamtlänge definieren und der proximale Abschnitt etwa 60 bis etwa 90% der Gesamtlänge beträgt, der Zwischenabschnitt etwa 15 bis etwa 20% der Gesamtlänge beträgt, und der distale Abschnitt etwa 2 bis etwa 7% der Gesamtlänge beträgt.

5. Katheterschaft nach einem der Ansprüche 2 oder 3, der ferner eine distale Spitze aufweist, die mit dem distalen Abschnitt des Katheterschafts gekoppelt ist.

6. Katheterschaft nach Anspruch 5, wobei die distale Spitze aus Polyetherpolyester besteht.

7. Katheterschaft nach Anspruch 2, wobei die Innenschicht Polytetrafluorethylen enthält.

8. Katheterschaft nach einem der Ansprüche 1 oder 2, wobei die Innenschicht hochdichtes Polyethylen enthält.

9. Katheterschaft nach einem der Ansprüche 1 oder 2, wobei das Stützelement ein Metallgeflecht aufweist.

10. Katheterschaft nach einem der Ansprüche 1 oder 2, wobei das Stützelement eine Wicklung aufweist.

11. Katheterschaft nach einem der Ansprüche 1 oder 2, der ferner eine distale Spitze aufweist, die mit der Innenschicht, der ersten Schicht und dem Stützelement gekoppelt ist und distal davon angeordnet ist.

12. Katheterschaft nach einem der Ansprüche 1 oder 2, wobei die Innenschicht ein Führungsdrahtlumen aufweist, das sich dort hindurch erstreckt.

13. Katheterschaft nach einem der Ansprüche 1 oder 2, wobei zwischen der Innenschicht und der ersten Schicht ein Inflationslumen definiert ist.

14. Verfahren zur Herstellung eines Katheterschafts nach Anspruch 1, das die Schritte aufweist:
Bereitstellen einer ersten Polymermischung, die etwa 80 bis etwa 95 Gew.-% Polyoxymethylen und etwa 5 bis etwa 20 Gew.-% Polyetherpolyester aufweist;
Bereitstellen einer zweiten Polymermischung, die etwa 20 bis etwa 50 Gew.-% Polyoxymethylen und etwa 50 bis etwa 80 Gew.-% Polyetherpolyester aufweist; und
Bereitstellen einer dritten Polymermischung, die etwa 5 bis etwa 20 Gew.-% Polyoxymethylen und etwa 80 bis etwa 95 Gew.-% Polyetherpolyester aufweist;
Extrudieren der ersten Polymermischung, um ein erstes Schaftelement zu definieren;
Extrudieren der zweiten Polymermischung, um ein zweites Schaftelement zu definieren;
Extrudieren der dritten Polymermischung, um ein drittes Schaftelement zu definieren; und
Koppeln des ersten Schaftelements, des zweiten Schaftelements und des dritten Schaftelements, um einen Katheterschaft zu definieren.

## Revendications

1. Tige de cathéter (20), comprenant :
une couche intérieure (30) ;
un élément de maintien disposé sur la couche intérieure (30) ; et
une couche extérieure (26) disposée sur la couche intérieure (30) et la couche de maintien, ladite couche extérieure comprenant :
une section proximale comportant 80 à 95 % en poids environ de polyoxyméthylène mélangé à 5 à 20 % en poids environ d'un polymère avec un groupe éther ;
une section distale comportant 5 à 20 % en poids environ de polyoxyméthylène et 80 à 95 % en poids environ d'un polymère avec un groupe éther ;
une section intermédiaire disposée entre la section proximale et la section distale, ladite section intermédiaire comportant 20 à 50 % en poids environ de polyoxyméthylène et 50 à 80 % en poids environ d'un polymère avec un groupe éther.

2. Tige de cathéter selon la revendication 1, où le polymère avec un groupe éther est du polyester-polyéther.

3. Tige de cathéter selon l'une des revendications 1 ou 2, où la section proximale a un module de flexion de 210 à 380 ksi environ ;
la section intermédiaire a un module de flexion de 30 à 90 ksi environ ; et où
la section distale a un module de flexion inférieur à 30 ksi.

4. Tige de cathéter selon l'une des revendications 2 ou 3, où la section proximale, la section intermédiaire et la section distale définissent une longueur totale de tige, et où la longueur de la section proximale est de 60 à 90% environ de la longueur totale, la longueur de la section intermédiaire est de 15 à 20% environ de la longueur totale, et la longueur de la section distale est de 2 à 7% environ de la longueur totale.

5. Tige de cathéter selon l'une des revendications 2 ou 3, comprenant en outre un embout distal raccordé à la section distale de la tige de cathéter.

6. Tige de cathéter selon la revendication 5, où l'embout distal est composé de polyester-polyéther.

7. Tige de cathéter selon la revendication 2, où la couche intérieure comprend du polytétrafluoroéthylène.

8. Tige de cathéter selon l'une des revendications 1 ou 2, où la couche intérieure comprend du polyéthylène haute densité.

9. Tige de cathéter selon l'une des revendications 1 ou 2, où l'élément de maintien comprend une tresse métallique.

10. Tige de cathéter selon l'une des revendications 1 ou 2, où l'élément de maintien comprend une bobine.

11. Tige de cathéter selon l'une des revendications 1 ou 2, comprenant en outre un embout distal raccordé et disposé distalement à la couche intérieure, à la première couche, et à l'élément de maintien.

12. Tige de cathéter selon l'une des revendications 1 ou 2, où la couche intérieure définit une lumière à fil guide s'étendant au travers de celle-ci.

13. Tige de cathéter selon l'une des revendications 1 ou 2, où une lumière de gonflement est définie entre la couche intérieure et la première couche.

14. Procédé de fabrication d'une tige de cathéter selon la revendication 1, comprenant les étapes de :
préparation d'un premier mélange polymère comportant 80 à 95 % en poids environ de polyoxyméthylène et 5 à 20 % en poids environ de polyester-polyéther ;
préparation d'un deuxième mélange polymère comportant 20 à 50 % en poids environ de polyoxyméthylène et 50 à 80 % en poids environ de polyester-polyéther ; et
préparation d'un troisième mélange polymère comportant 5 à 20 % en poids environ de polyoxyméthylène et 80 à 95 % en poids environ de polyester-polyéther ;
extrusion du premier mélange polymère pour définir un premier élément de tige ;
extrusion du deuxième mélange polymère pour définir un deuxième élément de tige;
extrusion du troisième mélange polymère pour définir un troisième élément de tige ; et
raccordement du premier élément de tige, du deuxième élément de tige et du troisième élément de tige pour définir une tige de cathéter.
